# EUROPÄISCHE PATENTSCHRIFT

(11) **EP 1 596 197 B1**
(45) Veröffentlichungstag und Bekanntmachung des Hinweises auf die Patenterteilung: **21.07.2010**
(21) Anmeldenummer: 05102547.6
(22) Anmeldetag: 31.03.2005
(51) Int. Cl.: G01N 33/20, C21B 7/24

(54) **Verfahren zur Ermittlung von mechanischen Eigenschaften von Metallen sowie Vorrichtung hierzu**
Method and device for determining the mechanical properties of metals
Méthode et appareil pour la determination des proprietes méchaniques de métaux

(30) Priorität: 14.05.2004 AT 8522004
(43) Veröffentlichungstag der Anmeldung: 16.11.2005
(73) Patentinhaber: Siemens VAI Metals Technologies GmbH & Co, 4031 Linz (AT)
(72) Erfinder: Chimani, Christian, 4540, Pfarrkirchen (AT); Bernhard, Christian, 8793, Trofaiach (AT); Tubikanec, Alexander, 4870, Vöcklamark (AT)
(74) Vertreter: Maier, Daniel Oliver

(56) Entgegenhaltungen:
- FR-A- 2 617 598
- US-A- 4 399 710
- US-A- 5 131 633

## Beschreibung

Die Erfindung betrifft ein Verfahren zur Ermittlung von mechanischen Eigenschaften, insbesondere Hochtemperatureigenschaften von Werkstoffen, vorzugsweise von Metallen, insbesondere von eisenhältigen Metallen, mit Gußstruktur, wie sie z.B. beim Stranggießen auftritt, wobei ein Probenträger in eine Schmelze des Werkstoffes getaucht und die am Probenträger erstarrte Schicht aus Schmelze auf Eigenschaften untersucht wird, sowie eine Vorrichtung zur Durchführung des Verfahrens.

Mechanische Hochtemperaturwerkstoffeigenschaften spielen bei der Beschreibung aller Prozesse eine Rolle, die eine Belastung bzw. Verformung des Werkstoffs bei hohen Temperaturen vorsehen. Dazu zählen bei der Stahlherstellung das Stranggießen, das Walzen und das Schweißen.

In Bezug auf das Stranggießen ist besonders die Duktilität des Werkstoffs und deren Abhängigkeit von Stahlzusammensetzung und Temperatur von Interesse, da man sich Aufschlüsse über die Bildung von Rissen erhofft. Die Festigkeit des Stahls bei hohen Temperaturen ist für die Auslegung von Stranggießmaschinen maßgeblich, die Bestimmung von Kriechgesetzen erlangt mit der Entwicklung der numerischen Simulation zunehmende Bedeutung.

Der überwiegende Teil der Laboruntersuchungen wird mit konventionellen Heißzugapparaturen durchgeführt, die eine induktive Erwärmung oder eine Widerstandserwärmung der Probe vorsehen. Von Schwerdtfeger stammt eine Zusammenfassung dieser Arbeiten (Schwerdtfeger, K.: Rißanfälligkeit von Stählen beim Stranggießen und Warmumformen, Verlag Stahleisen, Düsseldorf 1994), die unter anderem die mangelnde Umsetzbarkeit der Ergebnisse in die Praxis bemängelt. Unterschiedliche Versuchsbedingungen, wie Temperaturführung, Dehnrate und Probengeometrie, führen zu einer breiten Streuung der Ergebnisse. Besonders klar konnte dies für unterschiedliche Temperaturzyklen gezeigt werden: Bereits in den 1970er Jahren führten Wilber et al. (Wilber, G.A. et al.: The Effect of Thermal History and Composition on the Hot Ductility of Low Carbon Steel, Met. Trans. A, 6a (1975), 1727 - 1735) und Palmaers (Palmaers, A. : High Temperature Mechanical Properties of Steel as a Means for Controlling Continuous Casting, CRM, No. 53, November 1978, 23 - 31) vergleichende Untersuchungen an Proben durch, die vor dem Zugversuch auf Raumtemperatur abgekühlt und in verschiedenen Zyklen wiedererwärmt wurden, oder andererseits aus dem flüssigen Zustand "in-situ" abgekühlt und getestet wurden. Es zeigten sich deutliche Unterschiede in der jeweils ermittelten Brucheinschnürung, die durch die mehrmalige Umkristallisation und die daraus folgenden Unterschiede im Gefüge begründet wurden. Die Probleme in Hinblick auf die Verwendung der Ergebnisse für die Beschreibung des Stranggießprozesses wurden ausführlich von Lankford diskutiert (Lankford, W.T.: Some Considerations of Strength and Ductility in the Continuous Casting Process, Met. Trans, 3 (1972), 1331 ― 1357).

Aus diesem Grund wurden Versuche mit Stranggießrelevanz zunehmend "in-situ", also nach Aufschmelzen und Erstarren der Probe in einem Quarzglasröhrchen, durchgeführt. Durch radiales Anblasen der Oberfläche mit Stickstoff konnte auch eine normal auf die Hauptspannungsrichtung orientierte Kristallisationsrichtung erreicht werden (Hertel, J., H. Litterscheidt, U. Lotter und H. Pircher: Laboratory Simulation of Strand Shell Stresses and Strains during Continuous Casting, Thyssen Technische Berichte, Heft 1/91, 31-42). Die Wärmeabfuhr kann jedoch durch das Quarzglasröhrchen nicht beliebig erhöht werden.

Zu den Nachteilen der konventionellen Heißzugeinrichtungen ist zu zählen, dass die Bestimmung der Brucheinschnürung lediglich eine qualitative Aussage über Bereiche mit verminderter Zähigkeit gibt und keine Aussage liefert, bei welcher Belastung es bereits zu partieller Rissbildung kommt.

Dazu kommt, dass die produzierte Struktur nur eingeschränkt jener einer Stranggießschale entspricht. Qualitative Vergleiche zwischen Strukturparametern von Versuchsproben und stranggegossenem Material sind bisher nicht bekannt.

Für den Stranggießprozess sind jedoch besonders kritische Dehnungswerte von Interesse, weshalb andere Versuchsanordnungen entwickelt wurden, die sich in Zug- und Biegeversuche einteilen lassen (Bernhard, C.: Simulation der Innenrissbildung beim Stranggießen von Stahl, BHM, 145 (2000), 22 - 29). Diese ermöglichen jedoch ausschließlich Untersuchungen im ersten Zähigkeitsminimum nahe der Solidustemperatur.

Yamanaka und Mitautoren führten Zugversuche an einem erstarrenden zylindrischen Block mit einem Durchmesser 155 mm durch (Yamanaka, A., K. Nakajima, K. Yasumoto, H. Kawashima und K. Nakai: Nouvelle evaluation de la contrainte critique de formation des criques internes en coulee continue, La Revue de Metallurgie - CIT, 89 (1992), 627-633). Die Schalendicke zu Versuchsbeginn liegt bei etwa 40 mm und wird über Thermoelemente, die sich im Inneren des Blocks befinden, gemessen. Die erstarrende Schale wird Dehnungen von bis zu 4% ausgesetzt, die Dehnung wird optisch bestimmt. Nach dem Versuch werden Innenrisse durch Schwefelabdrücke detektiert. Matsumiya führte "in-situ Schmelz-Biege" - Versuche durch (Matsumiya, T., M. Ito, H. Kajioka, S. Yamaguchi und Y. Nakamura: An Evaluation of Critical Strain for Internal Crack Formation in Continuously Cast Slabs, Trans. ISIJ 26 (1986), 541-546). Bei dieser Versuchsanordnung wird eine Probe lokal aufgeschmolzen und gebogen. Die Dehnungen an der Phasengrenze fest/flüssig können jedoch nur berechnet werden. Die kritischen Dehnungswerte liegen - in Abhängigkeit der Stahlzusammensetzung - zwischen 1 und 4 %. Schmelz-Biegeversuche wurden vor allem von japanischen Forschern durchgeführt, eine Zusammenstellung der Untersuchungsergebnisse findet sich bei Nagata und Mitautoren (Nagata, S., T. Matsumiya, K. Ozawa und T. Ohashi: Estimation of Critical Strain for Internal Crack Formation in Continuously Cast Slab, Tetsu-to-Hagané 76 (1990), 214-221).

Von Wünnenberg und Flender (Wünnenberg, K. und R. Flender: Investigation of Internal-Crack Formation in Continuous Casting, Using a Hot Model, Ironmaking and Steelmaking 12-1 (1985), 22-29) wurde ein Block mit einer Höhe von 715 mm, einer Breite von 500 mm und einer Dicke von 200 mm vergossen, wobei eine Seite des Blocks von einer wassergekühlten Kupferplatte begrenzt wird, die nach etwa 3 min Erstarrungszeit entfernt wird. Danach drückt ein hydraulisch betriebener Stempel die erstarrte Schale nach innen und erzeugt an der Phasengrenze fest/flüssig eine definierte Dehnung. Wintz und Mitautoren (Wintz, M., M. Bobadilla und J.M. Jolivet: Hot cracking during solidification of steels: effect of carbon, sulphur and phosphorus, La Rev. de Met. - CIT, 91-1 (1994), 106-114) verwendeten für ihre Biegeversuche einen ähnlichen Versuchsaufbau.

Ein Nachteil der Biegeversuche ist, dass die auftretenden Dehnungen nur berechnet werden können, da sie für eine Messung nicht zugänglich sind. Die Qualität der Ergebnisse ist deshalb auch mit der Qualität der rechnerischen Beschreibung des Versuchs verbunden

Eine weiterer Versuchsaufbau zur Untersuchung von mechanischen Hochtemperatureigenschaften und der Rissanfälligkeit von Metallen stammt von Kurz und Mitautoren(Ackermann, P., W. Kurz und W. Heinemann: In Situ Tensile Testing of Solidifying Aluminium and Al-Mg-Shells, Mat. Science and Eng., 75 (1985), 79-86; Wagnieres, J.D. und P. Ackermann: Le laboratoire d'aujourd'hui pour les brames de demain, La Revue Politechnique, 6 (1985), 669-673). Sie führten mit der später so benannten SSCT (Submerged Split-Chill Tensile)-Apparatur Heißzugversuche an erstarrenden Al - Legierungen und Stahl durch. Die Apparatur wurde später vom Lehrstuhl für Metallurgie der Montanuniversität Leoben übernommen und adaptiert (Xia, G.: Untersuchungen über das mechanische Verhalten von erstarrendem Stahl unter stranggießähnlichen Bedingungen, Dissertation, Institut für Eisenhüttenkunde, Montanuniversität Leoben 1992; Xia, G., J. Zirngast, H. Hiebler und M. Wolf: High Temperature Mechanical Properties of in-situ Solidified Steel Measured by the SSCT-Test, Proceedings of the 1st Conference on Continuous Casting of Steel in Developing Countries, Beijing 1993, 200-210). Ein ähnliches Konzept wurde auch von japanischen Forschern angewendet (Suzuki, M., Suzuki, M., Yu, C. und T. Emi: In-situ Measurement of fracture strength of solidifying steel shells to predict upper limit of casting speed in continuous caster with oscillating mold, Trans. ISIJ 37 (1997), 375 - 382).

Grundprinzip dieser Versuche ist, dass ein zweigeteilter Prüfkörper aus Stahl in eine in einem Induktionsofen befindliche Stahlschmelze eintaucht. Der Prüfkörper kann mit keramischen Materialien beschichtet sein. Die Wärmeabfuhr wird über Art und Dicke der Beschichtung kontrolliert.

Während einer Haltezeit erstarrt an der Oberfläche eine Stahlschale. Die Hauptkristallisationsrichtung liegt entgegengesetzt der Richtung der Wärmeabfuhr, d.h. normal auf die Prüfkörperoberfläche. Nach Ende der Haltezeit wird im Induktionsofen der untere Prüfkörperteil hydraulisch abgesenkt und die Stahlschale auf Zug beansprucht. Während des Zugversuchs werden Kraft und verfahrener Weg aufgezeichnet. Die Erwärmung des Prüfkörpers wird mit Thermoelementen im Inneren aufgezeichnet. Aus den gemessenen Temperaturen lässt sich die Wärmestromdichte an der Prüfkörperoberfläche berechnen (Hiebler, H. und C. Bernhard: Mechanical Properties and Crack Susceptibility of Steel during Solidification, steel research 69-9 (1999), 349-355). Aus den gemessenen Temperaturen wird der Wärmeübergang zwischen Schale und Prüfkörper errechnet und daraus das Schalenwachstum und die Temperaturverteilung in der Strangschale.

Die metallografische Untersuchung der Probe nach dem Versuch gestattet eine Aussage über das Auftreten von Rissen und das Ausmaß der Rissbildung (Hiebler, H und C. Bernhard: Mechanical Properties and Crack Susceptibility of Steel during Solidification, steel research 69-9 (1999), 349-355). Verknüpft mit veränderbaren Versuchsbedingungen können daraus Aussagen über kritische Belastungen und den Einfluss von Legierungselementen getroffen werden. Vorteile der Methode sind nach Bernhard (Bernhard, C.: Simulation der Innenrissbildung beim Stranggießen von Stahl, BHM, 145 (2000), 22 - 29):
- Struktur und Gefüge entsprechen jener einer stranggegossenen Schale;
- die Belastung erfolgt normal auf die Hauptkristallisationsrichtung, also unter stranggießähnlichen Bedingungen;
- Wärmeabfuhr ist innerhalb des Bereichs konventioneller und endabmessungsnaher Gießverfahren veränderbar;
- Rissbildung kann quantifiziert und den Versuchsbedingungen oder der Stahlzusammensetzung zugeordnet werden;
- der Aufwand zur Durchführung von Versuchen ist vergleichsweise gering.

Nachteilig hierbei ist eine Änderung der Schalendicke während des Versuches, wenn dieser eine bestimmte Zeitspanne überschreitet. Die Versuchszeit ist prinzipiell nach oben begrenzt, sodass auch hierbei ausschließlich Untersuchungen im ersten Zähigkeitsminimum nahe der Solidustemperatur des untersuchten Werkstoffes durchgeführt werden können.

Aus der Schrift US 5 131 633 ist eine Vorrichtung zur Entnahme einer Probe einer Schlacke aus einer flüssigen Stahlschmelze bekannt, wobei die Vorrichtung einen Schmelztiegel zur Aufnahme einer Schmelze und einen in den Schmelztiegel mittels einer Taucheinrichtung eintauchbaren sowie herausnehmbaren Probenträger aufweist.

Zusammenfassend kann gesagt werden, dass während der letzten Jahrzehnte zahlreiche Anstrengungen unternommen wurden, einen prozessnahen Laborversuch zur Simulation der Belastung einer erstarrenden Strangschale beim Stranggießen zu entwickeln. Konventionelle Heißzugversuche weisen das Manko der mangelnden Übereinstimmung der Mikrostruktur der Probe mit jener eines Gussprodukts auf. Die Belastung der Probe bis zum vollständigen Zerreißen liefert mit der Brucheinschnürung zwar einen qualitativen Hinweis auf die Rissempfindlichkeit, die Ableitung eines Verformungslimits ist jedoch schwierig.

Prozessnähere Zug- und Biegeversuche sind vergleichsweise aufwendig und auf den Bereich des ersten Zähigkeitsminimums beschränkt.

Die Erfindung bezweckt die Vermeidung dieser Nachteile und Schwierigkeiten und stellt sich die Aufgabe, ein Verfahren der einleitend beschriebenen Art sowie eine Vorrichtung zur Durchführung des Verfahrens zu schaffen, welche es ermöglichen, einen Werkstoff mit Gefüge- und Struktureigenschaften zu untersuchen, die dem Zustand nach dem Vergießen des Metalles entsprechen. Insbesondere soll eine beim Erstarren von kristallinen Werkstoffen, wie z.B. von Metallen, von den Abkühlbedingungen abhängige Kristallstruktur bzw. eine Umwandlung der Kristallstruktur Berücksichtigung finden.

Diese Aufgabe wird erfindungsgemäß dadurch gelöst, dass der Probenträger nach Anhaften einer Schicht von Schmelze aus der Schmelze entnommen und die am Probenträger haftende durcherstarrte Schicht von Schmelze hinsichtlich ihrer Eigenschaften im am Probenträger haftenden Zustand untersucht wird, vorzugsweise nach Abkühlung auf eine vorbestimmte Temperatur.

Wesentlich ist hierbei, daß die am Probenträger haftende Schicht von Schmelze während der Prüfung durcherstarrt ist und nicht mit noch flüssiger Schmelze in Kontakt steht. Hierdurch ist es möglich, die Untersuchung der mechanischen Eigenschaften dann durchzuführen, wenn die durcherstarrte Schicht von Schmelze den für die Untersuchung relevanten Temperaturbereich erreicht hat.

Vorzugsweise wird die am Probenträger haftende durcherstarrte Schicht von Schmelze auf Zug beansprucht, wobei deren Duktilität und/oder Kriechverhalten und/oder Bruchdehnung und/oder Zähigkeit und/oder Zugfestigkeit ermittelt wird.

Mit Hilfe des erfindungsgemäßen Verfahrens ist es auch möglich, an der am Probenträger anhaftenden Schicht das Schrumpfverhalten derselben zu ermitteln.

Um eine nähere Untersuchung von auftretenden Rissen bzw. Bruchstellen ohne Verzunderungen etc. zu ermöglichen, wird zweckmäßig nach dem Entnehmen des Probenträgers aus der Schmelze der Probenträger mit der daran erstarrten Schmelze vor Ermittlung deren Eigenschaften mit Inertgas umgeben.

Vorzugsweise werden mit dem erfindungsgemäßen Verfahren für eine Stahlschmelze Eigenschaften in einem Temperaturbereich der Austenit-Ferrit-Umwandlung bestimmt.

Eine Vorrichtung zur Durchführung des Verfahren mit einem Schmelztiegel zur Aufnahme einer Schmelze eines Werkstoffes, einem in den Schmelztiegel mittels einer Tauchvorrichtung eintauchbaren sowie heraushebbaren zweiteiligen Probenträger, dessen beide Teile mittels einer Krafteinrichtung voneinander bewegbar sind, sowie mit einem den Probenträger sowie dessen Betätigungseinrichtungen aufnehmenden Träger, ist dadurch gekennzeichnet, dass der Träger und der Schmelztiegel voneinander trennbar, insbesondere unter Durchführung einer seitlichen Relativbewegung auseinanderbewegbar sind.

Um Veränderungen an der am Probenträger anhaftenden Schicht von Schmelze während und/oder nach dem Prüfverfahren zu vermeiden, ist die erfindungsgemäße Vorrichtung zweckmäßig gekennzeichnet durch eine Inertgasschutzhaube, in die der Probenträger nach dem Überziehen mit erstarrter Schmelze einbringbar bzw. die über den Probenträger stülpbar ist.

Zur Ermittlung des Schrumpfverhaltens ist die Vorrichtung dadurch gekennzeichnet, dass die zwei Teile des Probenträgers beim oder vor dem Eintauchen in die Schmelze voneinander geringfügig trennbar sind, u.zw. unter Vermeidung des Eindringens von Schmelze zwischen die beiden Teile des Probenträgers, vorzugsweise unter Anwendung einer Lippendichtung und/oder einer Asbestdichtung.

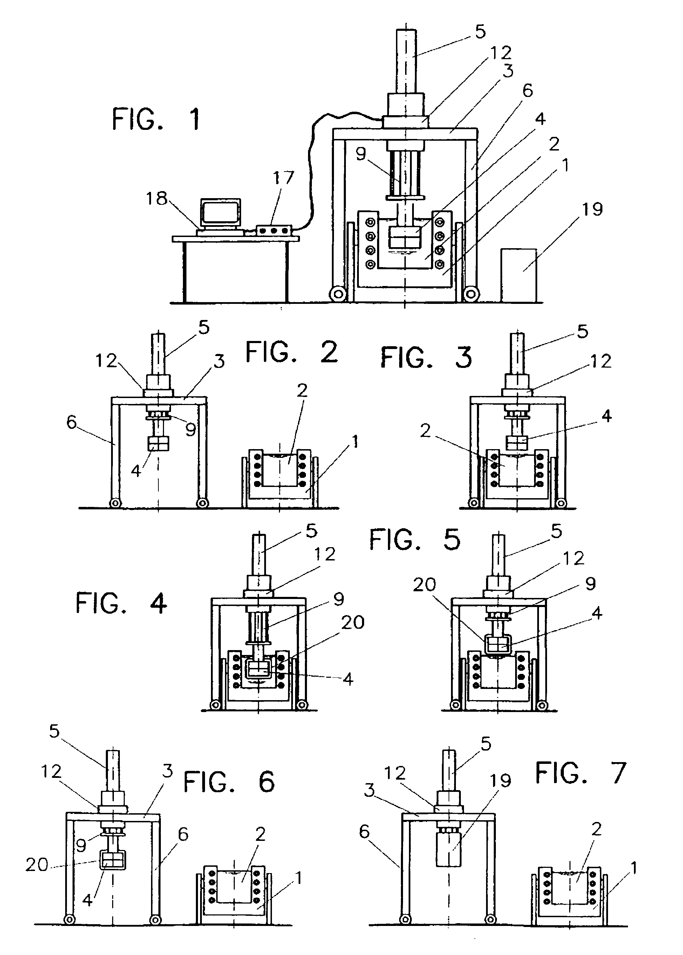

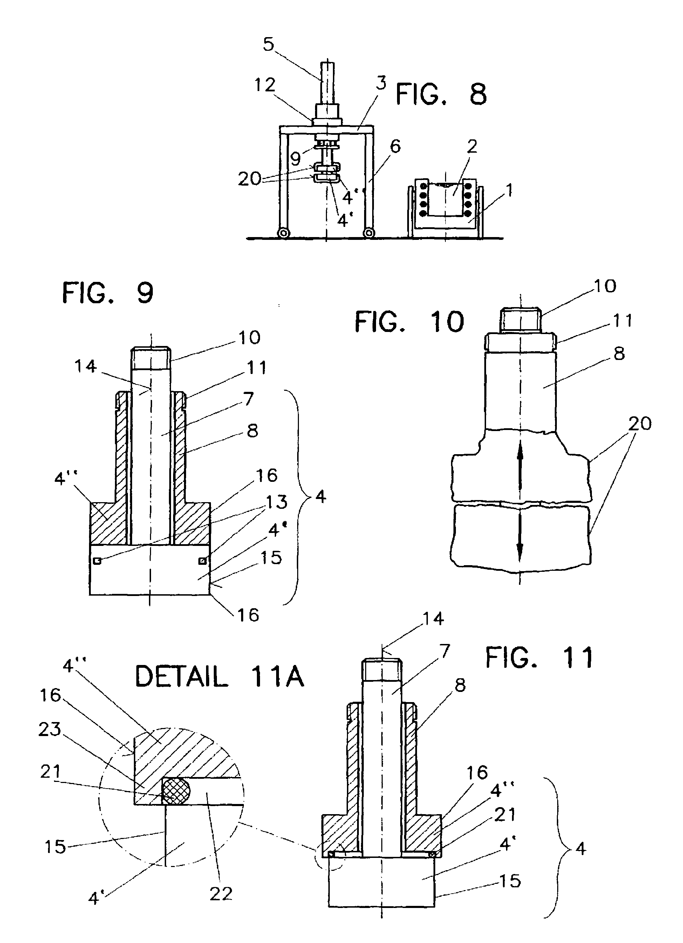

Die Erfindung ist nachfolgend anhand eines Ausführungsbeispieles näher erläutert, das in der Zeichnung schematisch dargestellt ist. Fig. 1 zeigt eine erfindungsgemäße Vorrichtung in Gesamtansicht; die Fig. 2 bis 8 veranschaulichen jeweils in zu Fig. 1 analoger Darstellung den Verfahrensablauf. Fig. 9 zeigt einen Probenträger im Schnitt und Fig. 10 denselben nach Durchführung einer Prüfung. Fig. 11 und Detail 11A zeigen eine abgewandelte Ausführungsform des Probenträgers zur Bestimmung des Schrumpfverhaltens von Werkstoffen.

Beim Erstarren von kristallinen Werkstoffen, wie zum Beispiel von Metallen, stellt sich eine von den Abkühlbedingungen abhängige Kristallstruktur ein. Für einen Großteil der industrierelevanten Gießprozesse gilt, dass die thermische Energie über die Bauteiloberflächen abgeführt wird. Unter Vernachlässigung von Strömungseinflüssen ergibt sich auf Grund der gerichteten Wärmeabfuhr ein Kristallwachstum, das der Wärmeabfuhrrichtung entgegengesetzt ist. Die dabei entstehende Struktur wird kolumnare Gussstruktur genannt. Dies trifft auch für die Gussstruktur eines beim Brammenstrangguss erstarrten Stahls zu.

Auf Grund dieser Ausrichtung des Kristallwachstums ergeben sich anisotrope mechanische Werkstoffeigenschaften, insbesondere der Werkstofffestigkeit und damit für das Werkstoffversagen, solange die Kristalle in ihrer Gussstruktur vorliegen. Erfolgt bei weiterer Abkühlung des Gussteils eine Phasenumwandlung, wie z.B. bei Stahl die Austenit-Ferrit-Umwandlung, so werden die Materialeigenschaften von der neu entstandenen Kristallstruktur und deren Phasen dominiert.

So z.B. erweist sich Stahl beim Vergießen auf konventionellen Stranggießanlagen als kritisch bezüglich seiner Oberflächenrissempfindlichkeit. In einem Temperaturbereich zwischen 800 und 1000°C führen schon geringe Dehnungen, wie sie z.B. beim Richten eines gekrümmten Strangs in einer Kreisbogenstranggießanlage auftreten, zur Bildung von Oberflächenrissen Aus der bestehenden Literatur sind qualitative Aussagen über die Auswirkung verschiedener Legierungs- und Begleitelemente auf die Duktilitätseigenschaften von Stahl bekannt. Eine Quantifizierung des Analyseneinflusses auf die Duktilitätseigenschaften ist jedoch nicht möglich. Weiters sind die Auswirkungen der bei den bekannten Untersuchungen zugrundeliegenden Versuchsbedingungen, wie z.B. Belastungsart und Temperaturführung, umstritten.

Im Rahmen der Erfindung ist es u.a. möglich, ausgewählte Stahlsorten mit Hilfe der in Fig. 1 dargestellten Vorrichtung im Temperaturbereich der Austenit-Ferrit-Umwandlung auf ihre Duktilitätseigenschaften zu untersuchen. Das erfindungsgemäße Verfahren erlaubt eine Untersuchung von Werkstoffen unter Randbedingungen, die den Prozessbedingungen des Stranggießverfahrens in Bezug auf Phasenanteile, Korngröße und der Beziehung zwischen Kornorientierung und Belastungsorientierung sehr nahe kommen und damit direkte Rückschlüsse auf das Werkstoffverhalten im Gießprozess erlauben.

Die in Fig. 1 dargestellte Vorrichtung weist als Schmelztiegel einen Induktionsofen 1 auf, in dem sich eine Metallschmelze 2, beispielsweise Stahlschmelze, befindet. An einem Träger 3 ist ein zweiteiliger Probenträger 4, wie er z.B. in den Fig. 9 und 11 dargestellt ist, befestigt. Dieser Probenträger 4 kann mittels einer Hub- und Senkeinrichtung 5, beispielsweise ausgebildet als hydraulischer Druckmittelzylinder, gehoben und gesenkt werden, wobei der Druckmittelzylinder 5 an dem Träger 3 montiert ist und mit diesem an einem aus dem Bereich des Induktionsofens 1 verfahrenbaren Rollwagen 6 montiert ist. Selbstverständlich kann der Rollwagen 6 auch durch eine andere Einrichtung, wie z.B. einen Kran od.dgl., ersetzt werden. Wesentlich ist, dass der Probenträger 4 nicht nur aus dem Induktionsofen 1 herausbewegt werden kann, sondern auch seitlich von ihm zur eigentlichen Ermittlung der mechanischen Eigenschaften des Metalles in Stellung gebracht werden kann.

Der Probenträger 4 selbst besteht aus zwei Teilen, u.zw. einem kolbenartigen Unterteil 4' und einem kolbenartigen Oberteil 4", wobei der Oberteil 4" gegenüber dem Unterteil 4' oder umgekehrt der Unterteil 4' gegenüber dem Oberteil 4" relativbeweglich ist, u.zw. zueinander und voneinander bewegbar. Das ist beispielsweise dadurch bewerkstelligbar, dass beide Teile Längsfortsätze 7, 8 aufweisen, wobei der Längsfortsatz 7 des Unterteiles 4' den Längsfortsatz 8 des Oberteiles 4" durchsetzt und mit einem die Relativbewegung zwischen Unter- und Oberteil verursachenden Druckmittelzylinder 9 verbunden ist, beispielsweise mit einem Gewindeendteil 10 mit dem Druckmittelzylinder 9 verschraubt ist.

Der Längsfortsatz 8 des Oberteiles 4" weist ebenfalls einen Gewindeendteil 11 auf, mit dem er ebenfalls mit dem Druckmittelzylinder 9 verschraubt ist. Durch Betätigen des Druckmittelzylinders 9 läßt sich somit die Relativbewegung zwischen Ober- 4" und Unterteil 4' durchführen.

Zur exakten Bestimmung der bei der Versuchsdurchführung auftretenden bzw. auf den Probenträger aufgebrachten bzw. auf ihn von Seiten des zu prüfenden Metalles einwirkenden Kräfte ist zweckmäßig eine Kraftmeßdose 12 vorgesehen.

Sowohl der Druckmittelzylinder 5 zum Auf- und Abbewegen des Probenträgers 4 als auch der Druckmittelzylinder 9 zur Aufbringung einer Kraft zwischen Ober- und Unterteil des Probenträgers 4 können durch Zahnstangen-, Gewindeantrieb-, Stellspindeln etc. ersetzt sein.

Die Querschnitte sowohl des Unterteiles 4' als auch des Oberteiles 4" des Probenträgers 4 sind vorzugsweise kreisförmig. Im Unterteil 4' sind Thermoelemente 13 untergebracht. Die Thermoelemente 13 befinden sich um die Längsachse 14 des Probenträgers 4 einander gegenüberliegend etwa 2 mm unter der Oberfläche 15 des Unterteiles 4'. Sie dienen dazu, die lokale Temperaturgeschichte bei Durchführung einer Prüfung zu erfassen. Als Thermoelemente 13 können z.B. Ni-Cr-Ni-Thermoelemente verwendet werden.

Sowohl der Oberteil 4" als auch der Unterteil 4' weisen ein- und denselben Außenquerschnitt auf und sie sind an ihrer Umfangsfläche mit einer Keramikschicht 16 beschichtet. Diese dient dazu, die Wärmeabfuhr zwischen dem Probenträger 4 und dem zu untersuchenden Metall zu regeln. Die Dicke der Keramikschicht 16 wird je nach Anwendungsfall und je nach zu prüfendem Metall gewählt. Ebenso sind die Abmessungen des Probenträgers 4 an die Versuchsart (Zug-, Kriech-, Relaxations- oder Schrumpfversuch) angepaßt.

Abhängig von den thermischen und mechanischen Prüfanforderungen kann der Probenträger 4 z.B. aus Ni-legiertem oder warmfestem Stahl hergestellt sein.

In Fig. 1 ist weiters eine Meßeinrichtung 17 mit einem Rechner 18 veranschaulicht. Weiters zeigt Fig. 1 eine an eine Inertgaszuleitung anschließbare Inertgas-Schutzhaube 19, in die der Probenträger 4 einbringbar ist bzw. die über den Probenträger 4 stülpbar ist.

Anhand der Fig. 2 bis 8 ist nachfolgend ein Versuchsablauf erläutert:

Zunächst wird in dem Induktionsofen 1 eine Schmelze 2 vorbereitet (Fig. 2), worauf der Probenträger 4 montiert wird und die Versuchsparameter eingestellt werden. Hierbei befindet sich der Rollwagen 6 noch seitlich des Induktionsofens 1. Sodann wird der Rollwagen 6 über dem Induktionsofen 1 in Stellung gebracht (Fig. 3) und der Probenträger 4 in die Schmelze 2 eingetaucht (Fig. 4). Nach kurzer Zeit bildet sich an der Oberfläche des Probenträgers 4 ein Prüfkörper 20, gebildet von einer am Probenträger 4 erstarrten Schicht der Schmelze 2, worauf der Probenträger 4 mit dem Prüfkörper 20 aus dem Induktionsofen 1 herausgehoben wird (Fig. 5) und seitlich des Induktionsofens 1 mit Hilfe des Rollwagens 6 verbracht wird (Fig. 6).

Anschließend wird über den Prüfkörper 20 die Inertgas-Schutzhaube 19 gestülpt, und es kann der Versuch, beispielsweise ein Zugversuch, durchgeführt werden (Fig. 7). Nach Ende des Zugversuches und ausreichendem Abkühlen des Prüfkörpers 20 wird die Inertgas-Schutzhaube 19 entfernt (Fig. 8), und es können gegebenenfalls nach Demontage des Probenträgers 4 die Bruchflächen bzw. gegebenenfalls entstandenen Oberflächenrisse,- das Gefüge etc. näher untersucht werden.

Figur 11 zeigt eine Ausführungsform des Probenträgers 4 für die Duchführung eines Schrumpfversuches. Hierbei ist der Unterteil 4'gegen den Oberteil 4" verschiebbar, und zwar durch das Schrumpfverhalten der am Probenträger 4 haftenden Schicht von Schmelze 2. Um eine einwandfreie Funktion des Gegeneinanderverschiebens der beiden Teile 4' und 4" zu gewährleisten, ist zwischen dem Oberteil 4" und dem Unterteil 4' eine Dichtschnur 21 aus Asbest eingelegt, die in einer Ausnehmung 22 des Oberteils 4" untergebracht ist. Zur Lagesicherung dieser Dichtschnur 21 übergreift der Oberteil 4" die Dichtschnur 21 peripher mit einer Art Dichtlippe 23.

Mit der erfindungsgemäßen Vorrichtung kann man eine Vielzahl von Versuchen unter stets gleichen Bedingungen unmittelbar nacheinander durchführen, wobei die chemische Zusammensetzung der Schmelze 2 zwischen den einzelnen Versuchen durch Legieren verändert werden kann. Die Vorrichtung kann für verschiedenartige Materialien, wie z.B. auch für Aluminiumguss, Magnesiumguss, Sonderlegierungen etc. Verwendung finden.

Zur Veranschaulichung des Versuchsablaufes sind nachfolgend beispielhaft die Versuchsparameter eines konkret durchgeführten Versuches aufgelistet. Die Versuche lassen sich in verschiedenster Art und Weise durchführen. Die Auswahl der Versuchsparameter wird an die zu untersuchenden Werkstoffdaten angepasst. Die wiedergegebenen Parameter stellen nur eine der vielen Möglichkeiten dar.

### Versuchsparameter:

• Chemische Analyse der Schmelze 2: (Angaben in Massenprozent)

| | |
|---|---|
| C | 0,142 % |
| Si | 0,350 % |
| Mn | 1,340 % |
| Al | 0,042 % |
| Ni | 0,224 % |
| Nb | 0,0256 % |
| N | 70 ppm |
| Fe | Rest |

• Schmelztemperatur: 1550 °C
• Liquidustemperatur: 1515 °C
• Stahlschmelze im Induktionsofen 1:
- Durchmesser: 130 mm
- Badtiefe: 300 mm

• Thermoelemente 13 Innen: 2 Stück Ni-Cr-Ni-Thermoelemente
• Thermoelemente 13 Außen: 1 Stück Pt-Pt-Rh-Thermoelement
• Eintauchzeit des Probenträgers 4: 2 sek
• Eintauchtiefe des Probenträgers 4: 95 mm
• Haltezeit im Induktionsofen 1: 1 sek
• Austauchzeit: 2 sek
• Inertgasspülung: Ar
• Inertgasmenge: 50l/min
• mittlere anerstarrte Schalenstärke des Prüfkörpers 20: 4,2 mm
• Haltezeit bis zum Beginn des Zugversuches: 20 sek.
• Versuchstemperatur:
- Außen:
   Zugversuchstarttemperatur: 789 °C
   Zugversuchendtemperatur: 687 °C
- Innen:
   Zugversuchstarttemperatur: 560 °C
   Zugversuchendtemperatur: 580 °C

• Zugversuch:
Dauer: 16 sek
Dehnrate: 0,3 mm/sek
Gesamtdehnungsweg: 4,8 mm
Maximalkraft: 26,35 kN

• Rissbildung: Die anerstarrte Stahlschale ist bei diesem Versuch vollständig durchgerissen. Der Riss verlief entlang der Teilungsebene des Probenträgers 4 normal zur Zugrichtung.

## Patentansprüche

1. Verfahren zur Ermittlung von mechanischen Eigenschaften von Metallen mit Gussstruktur, wobei ein Probenträger (4) in eine Schmelze (2) des Metalls getaucht, der Probenträger (4) nach Anhaften einer Schicht von Schmelze (2) aus der Schmelze (2) entnommen und die am Probenträger (4) erstarrte Schicht aus Schmelze (2) auf Eigenschaften untersucht wird, **dadurch gekennzeichnet, dass** nachdem der Probenträger (4) entnommen wurde, die am Probenträger (4) haftende durcherstarrte Schicht von Schmelze (2) hinsichtlich ihrer mechanischen Eigenschaften im am Probenträger (4) haftenden Zustand untersucht wird.

2. Verfahren nach Anspruch 1, **dadurch gekennzeichnet, dass** die am Probenträger (4) haftende durcherstarrte Schicht von Schmelze (2) nach Abkühlung auf eine vorbestimmte Temperatur hinsichtlich ihrer mechanischen Eigenschaften im am Probenträger (4) haftenden Zustand untersucht wird.

3. Verfahren nach einem der Ansprüche 1 oder 2, **dadurch gekennzeichnet, dass** die am Probenträger (4) haftende durcherstarrte Schicht von Schmelze (2) auf Zug beansprucht wird, wobei deren Duktilität und/oder Kriechverhalten und/oder Bruchdehnung und/oder Zähigkeit und/oder Zugfestigkeit ermittelt wird.

4. Verfahren nach einem der Ansprüche 1 bis 3, **dadurch gekennzeichnet, dass** an der am Probenträger (4) haftenden Schicht von Schmelze (2) das Schrumpfverhalten ermittelt wird.

5. Verfahren nach einem oder mehreren der Ansprüche 1 bis 4, **dadurch gekennzeichnet, dass** nach dem Entnehmen des Probenträgers (4) aus der Schmelze (2) der Probenträger (4) mit der daran erstarrten Schmelze vor Ermittlung deren Eigenschaften mit Inertgas umgeben wird.

6. Verfahren nach einem oder mehreren der Ansprüche 1 bis 5, **dadurch gekennzeichnet, dass** für eine Stahlschmelze (2) Eigenschaften in einem Temperaturbereich der Austenit-Ferrit-Umwandlung bestimmt werden.

7. Vorrichtung zur Durchführung des Verfahrens nach einem oder mehreren der Ansprüche 1 bis 6, mit einem Schmelztiegel (1) zur Aufnahme einer Schmelze (2) eines Werkstoffes, einem in den Schmelztiegel (1) mittels einer Tauchvorrichtung (5) eintauchbaren sowie heraushebbaren zweiteiligen Probenträger (4), **dadurch gekennzeichnet, dass** dessen beide Teile (4', 4") mittels einer Krafteinrichtung (9) voneinander bewegbar sind, sowie mit einem den Probenträger (4) sowie dessen Betätigungseinrichtungen (5, 9) aufnehmenden Träger (3), wobei der Träger (3) und der Schmelztiegel (1) voneinander trennbar, insbesondere unter Durchführung einer seitlichen Relativbewegung auseinanderbewegbar sind.

8. Vorrichtung nach Anspruch 7, **dadurch gekennzeichnet, dass** die Vorrichtung zur Vermeidung des Eindringens von Schmelze (2) zwischen die beiden Teile (4', 4") des Probenträgers (4) eine Lippendichtung (23) und/oder einer Asbestdichtung (21) aufweist.

9. Vorrichtung nach Anspruch 7 oder 8, **gekennzeichnet durch** eine Inertgas-Schutzhaube (19), in die der Probenträger (4) nach dem Überziehen mit erstarrter Schmelze einbringbar bzw. die über den Probenträger (4) stülpbar ist.

## Claims

1. Method for determining mechanical properties of metals having a cast structure, wherein a sample carrier (4) is dipped into a melt (2) of the metal, the sample carrier (4) is removed from the melt (2) after a layer of melt (2) has adhered to it, and the properties of the layer of melt (2) solidified on the sample carrier (4) are investigated, **characterized in that**, after the sample carrier (4) has been removed, the fully solidified layer of melt (2) adhering to the sample carrier (4) is investigated with regard to its mechanical properties in the state adhering to the sample carrier (4).

2. Method according to Claim 1, **characterized in that**, after it has been cooled to a predetermined temperature, the fully solidified layer of melt (2) adhering to the sample carrier (4) is investigated with regard to its mechanical properties in the state adhering to the sample carrier (4).

3. Method according to either of Claims 1 and 2, **characterized in that** a tensile load is applied to the fully solidified layer of melt (2) adhering to the sample carrier (4), the ductility and/or creep behaviour and/or elongation at break and/or toughness and/or tensile strength of said layer being determined.

4. Method according to one of Claims 1 to 3, **characterized in that** the shrinkage behaviour is determined on the layer of melt (2) adhering to the sample carrier (4).

5. Method according to one or more of Claims 1 to 4, **characterized in that**, after the sample carrier (4) has been removed from the melt (2), the sample carrier (4) with the melt solidified thereon is surrounded with inert gas before the properties thereof are determined.

6. Method according to one or more of Claims 1 to 5, **characterized in that** properties of a steel melt (2) are determined in a temperature range of austenite-ferrite conversion.

7. Apparatus for carrying out the method according to one or more of Claims 1 to 6, having a melting crucible (1) for holding a melt (2) of a material, a two-part sample carrier (4) which can be dipped into and lifted out from the melting crucible (1) by means of a dipping apparatus (5), **characterized in that** the two parts (4', 4") of said sample carrier can be moved apart by means of a force device (9), and having a carrier (3) which holds the sample carrier (4) and the actuating devices (5, 9) thereof, wherein the carrier (3) and the melting crucible (1) can be separated from each other, in particular can be moved apart by carrying out a lateral relative movement.

8. Apparatus according to Claim 7, **characterized in that** the apparatus for preventing the penetration of melt (2) between the two parts (4', 4") of the sample carrier (4) has a lip seal (23) and/or an asbestos seal (21).

9. Apparatus according to Claim 7 or 8, **characterized by** an inert gas protective hood (19), into which the sample carrier (4) can be introduced after it has been coated with solidified melt or which can be placed over the sample carrier (4).

## Revendications

1. Procédé de détermination des propriétés mécaniques de métaux à structure de coulée, dans lequel
un porte-échantillon (4) est immergé dans un bain de fusion (2) du métal,
le porte-échantillon (4) est retiré du bain de fusion (2) après qu'une couche de bain de fusion (2) y a adhéré et
les propriétés de la couche de bain de fusion (2) qui s'est solidifiée sur le porte-échantillon (4) sont étudiées, **caractérisé en ce que**
après que le porte-échantillon (4) a été retiré, les propriétés mécaniques de la couche de bain de fusion (2) durcie et adhérant au porte-échantillon (4) sont étudiées dans son état adhérant au porte-échantillon (4).

2. Procédé selon la revendication 1, **caractérisé en ce que** les propriétés mécaniques de la couche de bain de fusion (2) solidifiée et adhérant au porte-échantillon (4) sont étudiées dans son état adhérant au porte-échantillon (4) après refroidissement à une température définie.

3. Procédé selon l'une des revendications 1 ou 2, **caractérisé en ce que** la couche de bain de fusion (2) solidifiée et adhérant au porte-échantillon (4) est sollicitée en traction et **en ce que** sa ductilité et/ou son comportement de fissuration et/ou son allongement à la rupture et/ou sa ténacité et/ou sa résistance en traction sont déterminées.

4. Procédé selon l'une des revendications 1 à 3, **caractérisé en ce que** le comportement de retrait de la couche de bain de fusion (2) adhérant au porte-échantillon (4) est déterminé.

5. Procédé selon l'une ou plusieurs des revendications 1 à 4, **caractérisé en ce qu'**après que le porte-échantillon (4) a été retiré du bain de fusion (2), le porte-échantillon (4) sur lequel le bain de fusion s'est solidifié est englobé dans un gaz inerte avant détermination de ses propriétés.

6. Procédé selon l'une ou plusieurs des revendications 1 à 5, **caractérisé en ce que** pour un bain de fusion (2) d'acier, on détermine les propriétés dans la plage de température de la transformation austénite-ferrite.

7. Dispositif en vue de l'exécution du procédé selon l'une ou plusieurs des revendications 1 à 6, et présentant
un creuset de fusion (1) qui reprend un bain de fusion (2) d'un matériau,
un porte-échantillon (4) en deux parties qui peut être plongé et extrait du creuset de fusion (1) au moyen d'un dispositif d'immersion (5),
**caractérisé en ce que**
les deux parties (4', 4") du porte-échantillon peuvent être écartées l'une de l'autre par un dispositif de force (9) avec un support (3) qui reprend le porte-échantillon (4) ainsi que ses dispositifs d'actionnement (5, 9) et
**en ce que** le support (3) et le creuset de fusion (1) peuvent être séparés l'un de l'autre et en particulier peuvent être écartés l'un de l'autre en étant déplacés latéralement l'un par rapport à l'autre.

8. Dispositif selon la revendication 7, **caractérisé en ce que** pour éviter que le bain de fusion (2) pénètre entre les deux parties (4', 4") du porte-échantillon (4), le dispositif présente un joint d'étanchéité (23) à lèvres et/ou un joint d'étanchéité (21) en amiante.

9. Dispositif selon les revendications 7 ou 8, **caractérisé par** un capot de protection à gaz inerte de protection (19) dans lequel le porte-échantillon (4) peut être placé et/ou qui peut être posé au-dessus du porte-échantillon (4) après qu'il a été recouvert par du bain de fusion solidifié.
